(19) Europäisches Patentamt
European Patent Office
Office européen des brevets

(11) Veröffentlichungsnummer: **0 163 926**
**B1**

(12) # EUROPÄISCHE PATENTSCHRIFT

(45) Veröffentlichungstag der Patentschrift: **12.07.89**

(51) Int. Cl.[4]: **C 07 D 209/28**

(21) Anmeldenummer: **85105194.6**

(22) Anmeldetag: **29.04.85**

(54) **Verfahren zur Herstellung von Acemetacin.**

(30) Priorität: **08.05.84 DE 3416895**

(43) Veröffentlichungstag der Anmeldung: **11.12.85 Patentblatt 85/50**

(45) Bekanntmachung des Hinweises auf die Patenterteilung: **12.07.89 Patentblatt 89/28**

(84) Benannte Vertragsstaaten: **AT BE CH DE FR GB IT LI NL SE**

(56) Entgegenhaltungen:
**EP-A-0 084 127**
**EP-A-0 087 654**
**EP-A-0 087 655**
**EP-A-0 126 942**

**Die Akte enthält technische Angaben, die nach dem Eingang der Anmeldung eingereicht wurden und die nicht in dieser Patentschrift enthalten sind.**

(73) Patentinhaber: **BAYER AG, D-5090 Leverkusen 1 Bayerwerk (DE)**

(72) Erfinder: **Samaan, Samir, Dr., Claudiusweg 9, D-5600 Wuppertal 1 (DE)**
Erfinder: **Horstmann, Harald, Dr., Claudiusweg 19, D-5600 Wuppertal 1 (DE)**

**EP 0 163 926 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft ein Verfahren zur Herstellung von Acemetacin.

Ein Verfahren zur Herstellung von Acemetacin entsprechend Formel I

(I)

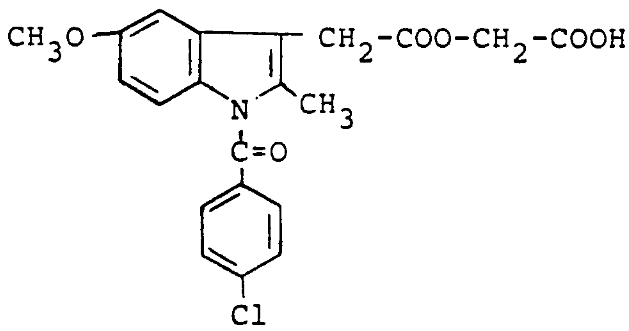

(I)

wird beispielsweise in der DE-OS-3 206 885 beschrieben. Die technische Durchführung des Verfahrens ist jedoch nicht ohne Probleme.

Es wurde ein neues Verfahren zur Herstellung von Acemetacin gefunden, dadurch gekennzeichnet, daß man Indometacine der allgemeinen Formel II

(II)

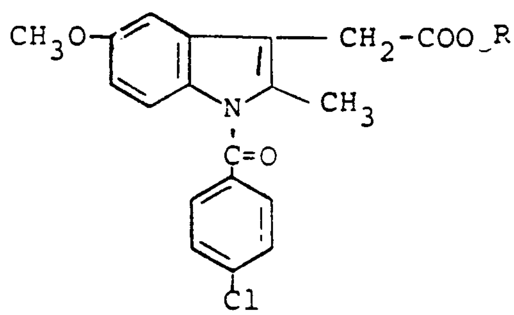

(II)

in der

R für ein Alkali- oder Erdalkalimetallkation oder für die Gruppe $MY_4$ steht

in der

M für Stickstoff oder Phosphor steht und

Y die Bedeutung Wasserstoff, gleiches oder verschiedenes, gegebenenfalls substituiertes Alkyl, Cycloalkyl oder Aryl hat,

mit Halogenessigsäure-tert.-butylester der allgemeinen Formel III

$Hal-CH_2-COO-C(CH_3)_3$ (III)

in der

Hal für Chlor, Brom oder Jod steht,

in Gegenwart eines Phasentransferkatalysators und von inerten, organischen Lösungsmitteln oder Gemischen davon im Temperaturbereich von -30°C bis +70°C umsetzt und anschließend mit katalytischen bis molaren Mengen von Schwefelsäure und/oder Sulfonsäuren im Temperaturbereich von -30 bis +120°C die weitere Umsetzung durchführt.

Nach dem erfindungsgemäßen Verfahren läßt sich Acemetacin besonders vorteilhaft herstellen.

Indometacine für das erfindungsgemäße Verfahren sind an sich bekannt.

Der erste Teil der erfindungsgemäßen Umsetzung wird im allgemeinen im Temperaturbereich von -30 bis +70° C, bevorzugt 0 bis 60° C, durchgeführt.

Sulfonsäuren können beispielsweise Methansulfonsäuren oder p-Toluolsulfonsäure sein.

Es können selbstverständlich auch Mischungen von Schwefelsäuren und der Sulfonsäuren eingesetzt werden.

Die Schwefelsäuren bzw. die Sulfonsäuren werden in katalytischen bis molaren Mengen eingesetzt

Bevorzugt setzt man 1 bis 50 % der molaren Menge ein.

Die Ausgangsverbindungen für das erfindungsgemäße Verfahren (allgemeine Formel II und III) werden im allgemeinen in etwa molaren Mengen umgesetzt.

Als Lösungsmittel für die erfindungsgemäße Reaktion kommen polare und aprotische Lösungsmittel wie Chloroform, Dichlormethan, Toluol, Aceton, Butanon-2 und Methylisobutylketon infrage. Für die Spaltungsreaktion sind auch Säuren wie Essigsäure als Lösungsmittel möglich. Bevorzugte Lösungsmittel sind Aceton, Essigsäure und Toluol.

Besonders bevorzugte Lösungsmittel bei der Reaktion zum tert.-Butylester sind Butanon-2, Aceton und Methylisobutylketon.

Die Verbindungen der allgemeinen Formel II liegen als Kalium-, Natrium-, quartäre Ammonium- oder Phosphoniumsalze vor.

Die erste Stufe des erfindungemäßen Verfahrens wird in Gegenwart eines Phasentransferkatalysators durchgeführt. Phasentransferkatalysatoren für das erfindungsgemäße Verfahren sind bevorzugt Ammonium- und/oder Phosphoniumsalze.

Als Phasentransferkatalysatoren sind besonders bevorzugt: Benzyltriethylammoniumchlorid, Tetrabutylammoniumbromid, -hydrogensulfat, Methyltrioctylammoniumchlorid, Tetrabutylphosphoniumbromid und Hexadecyltributylphosphoniumbromid.

Das erfindungsgemäße Verfahren kann in zwei Stufen aber auch als Eintopfverfahren, also ohne Isolierung des Zwischenproduktes, durchgeführt werden. Besonders bevorzugt wird die Ausführungsform des erfindungsgemäßen Verfahrens ohne Isolierung des Zwischenproduktes.

Wenn beispielsweise die erfindungsgemäße Umsetzung in Aceton als Lösungsmittel und mit Benzyltriethylammoniumchlorid als Phasentransferkatalysator (PTK) durchgeführt wird, so kann das Verfahren durch folgendes Schema wiedergegeben werden:

$$\text{II} \xrightarrow[\text{3) } H_2SO_4 \quad \text{4) } H_2O]{\text{1) } K_2CO_3\text{/Aceton, PTK} \quad \text{2) } Cl\text{-}CH_2COO\text{-}C(CH_3)_3} \text{I}$$

Hierbei wird das Zwischenprodukt nicht isoliert. Falls eine Isolierung des Acemetacin-tert. butylester erwünscht ist, so wird anstelle der Säure Wasser zugegeben, wodurch der Ester in hoher Reinheit und 95 % Ausbeute auskristallisiert.

Es ist bekannt tert.-Butylester mit Trifluoressigsäure oder Ameisensäure selektiv zu spalten (vgl. z. B.: T.W. Greene, Protective Groups in Organic Synthesis, Wiley & Sons, New York 1981, pp. 168). Diese Reagenzien sind jedoch relativ teuer und ihre Handhabung im technischen Maßstab erfordert besondere Sicherheitsmaßnahmen. Die Verwendung von wäßrigen Mineralsäuren wie Salzsäure, Bromwasserstoffsäure oder Schwefelsäure zur Spaltung des Acemetacin-tert.-butylesters mit hoher Selektivität zu Acemetacin hat sich als ungeeignet erwiesen. Entweder ist der Ester stabil (z. B. in 50-%-iger Schwefelsäure bei 25 - 30°C) oder die Spaltung ist unselektiv, d.h. es entsteht bis zu 60 % die Ausgangsverbindung Indometacin.

Erfindungsgemäß gelingt es überraschend den Acemetacinter.-butylester mit katalytischen bis molaren Mengen konz. Schwefelsäure oder Sulfonsäuren (96 - 98-%-ig) zu Acemetacin mit hoher Ausbeute und Reinheit zu spalten. Das erfindungsgemäße Verfahren hat den besonderen Vorteil der einfachen Aufarbeitung, die darin besteht, durch Wasserzugabe das Acemetacin auszukristallisieren und zu filtrieren.

Acemetacin ist ein bekanntes Antiphlogistikum in der Human- und Veterinärmedizin.

**Beispiel 1** (Acemetacin-tert.-butylester)

50,0 g Indometacin und 20,0 g Kaliumcarbonat in 280 ml Aceton werden 30 Minuten bei 56°C gerührt. Es wird auf 40°C abgekühlt, 1,0 g Benzyltriethylammoniumchlorid zugegeben und 25,3 g Chloressigsäure-tert.-butylester innerhalb 30 Minuten zugetropft. Es wird solange bei 40°C nachgerührt bis die Umsetzung beendet ist (5 bis 6 Stunden, DC-Kontrolle). Nach beendeter Umsetzung wird auf 20°C abgekühlt und auf 1 l Wasser gegossen. Es wird abgesaugt, mit Wasser gut gewaschen und im Vakuum bei 50°C getrocknet.

Ausbeute: 63,6 g ≙ 96 % der Theorie
Schmelzpunkt: 100 bis 101°C
Gehalt nach HPLC: 98,1 %.

**Beispiel 2** (Acemetacin)

10 g Acemetacin-tert.-butylester nach Beispiel 1 in 80 ml Toluol werden bei 45°C mit 0,4 ml Methansulfonsäure versetzt und 4 Stunden bei dieser Temperatur gerührt. Es werden weitere 0,2 ml Methansulfonsäure zugegeben und weitere 4 Stunden bei 45°C gerührt. Es wird abgekühlt und mit n-Hexan ausgefällt. Es wird abgesaugt, mit Wasser gewaschen, getrocknet und noch einmal aus Toluol n-Hexan umkristallisiert.
Ausbeute: 7,9 g ≙ 89,7 % der Theorie.

**Beispiel 3** (Acemetacin-Eintopfverfahren)

25 g Indometacin, 10 g Kaliumcarbonat und 190 ml Aceton werden 30 Minuten bei 56°C gerührt. Es wird auf 40°C abgekühlt, 0,5 g Benzyl-triethylammoniumchlorid zugegeben und 15,6 g Chloressigsäure-tert.-butylester zugetropft. Nach 5 Stunden bei 40°C ist die Reaktion beendet (DC-Kontrolle). Es wird auf 10°C abgekühlt, dann tropft man 20 ml konz. Schwefelsäure so zu, daß die Temperatur 25°C nicht überschreitet. Es wird 6 Stunden bei 20 bis 25°C gerührt (DC-Kontrolle), mit Wasser versetzt (ca. 500 ml), abgesaugt, im Vakuum getrocknet und aus Aceton-Wasser umkristallisiert.
Ausbeute: 26,7 g ≙ 91,9 % der Theorie.

**Beispiel 4** (Acemetacin)

15,0 g Acemetacin-tert.-butylester nach Beispiel 1 werden bei 20 - 25°C in 90 ml Eisessig suspendiert und mit 1,5 ml konz. Schwefelsäure versetzt. Nach ca. 30 Minuten entsteht eine klare Lösung. Nach weiteren 30 Minuten beginnt das Acemetacin auszukristallisieren. Es wird insgesamt 6 Stunden gerührt bis die Spaltung beendet ist (DC-Kontrolle). Es wird mit 100 ml Wasser versetzt, abgesaugt, mit Wasser gut gewaschen und im Vakuum bei 50°C getrocknet. Ausbeute: 13,2 g (95,2 % der Theorie).

**Beispiel 5** (Acemetacin)

9,6 g Acemetacin-tert.-butylester nach Beispiel 1 in 30 ml Essigsäure werden mit 0,4 g p-Toluolsulfonsäure versetzt und 4 bis 5 Stunden bei 100°C gerührt. Nach Abkühlung auf Raumtemperatur werden 50 ml Wasser zugegeben. Das Acemetacin fällt aus, wird abgesaugt und aus Aceton/Wasser umkristallisiert.
Ausbeute: 8,0 g ≙ 90,6 g der Theorie Reinprodukt.

**Patentansprüche**

1. Verfahren zur Herstellung von Acemetacin, dadurch gekennzeichnet, daß man ein Indometacin der allgemeinen Formel II

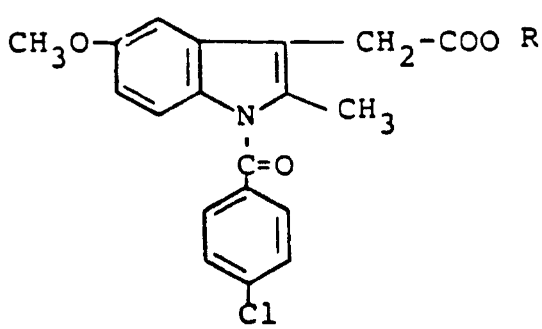

(II)

in der
R für ein Alkali- oder Erdalkalimetallkation oder für die Gruppe $MY_4$ steht,
in der
M für Stickstoff oder Phosphor steht und Y die Bedeutung Wasserstoff, gleiches oder verschiedenes,

gegebenenfalls substituiertes Alkyl, Cycloalkyl oder Aryl hat,
mit einem Halogenessigsäure-tert.-butylester der allgemeinen Formel III

$Hal-CH_2-COO-C(CH_3)_3$ (III)

in der
Hal für Chlor, Brom oder Jod steht,
in Gegenwart eines Phasentransferkatalysators und von inerten, organischen Lösungsmitteln oder Gemischen davon im Temperaturbereich von -30°C bis +70°C umsetzt und anschließend mit katalytischen bis molaren Mengen von Schwefelsäure und/oder Sulfonsäure im Temperaturbereich von -30°C bis +120°C die weitere Umsetzung durchführt.

2. Verfahren nach Anspruch 1, dadurch gekennzeichnet, daß die Umsetzung als Eintopfreaktion geführt wird.

3. Verfahren nach den Ansprüchen 1 bis 2, dadurch gekennzeichnet, daß man als Phasentransferkatalysator Benzyl-triethyl-ammoniumchlorid verwendet.

4. Verfahren nach den Ansprüchen 1 und 3, dadurch gekennzeichnet, daß man Acemetacin-tert.-butylester als Zwischenprodukt isoliert und dann die Spaltung durchführt.

5. Verfahren nach den Ansprüchen 1 bis 4, dadurch gekennzeichnet, daß die Spaltung zum Acemetacin mit katalytischen bis molaren Mengen Schwefelsäure in Eisessig oder Aceton oder in Gemischen dieser Lösungsmittel durchgeführt wird.

6. Verfahren nach den Ansprüchen 1 bis 5, dadurch gekennzeichnet, daß die Spaltung mit Sulfonsäuren in essigsauren oder organischen Lösungsmitteln durchgeführt wird.

**Claims**

1. Process for preparing acemetacin, characterised in that an indometacin of the general formula II

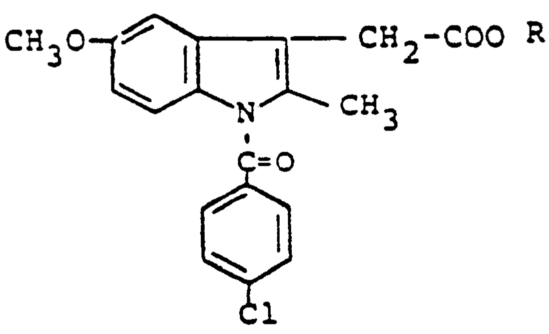

(II)

in which
R stands for an alkali metal or alkaline earth metal cation or for the group $MY_4$,
in which
M stands for nitrogen or phosphorus and
Y has the meaning hydrogen, identical or different, optionally substituted alkyl, cycloalkyl or aryl, is reacted with a tert.-butyl halogenoacetate of the general formula III

$Hal-CH_2-COO-C(CH_3)_3$ (III)

in which Hal stands for chlorine, bromine or iodine, within the temperature range from -30°C to +70°C in the presence of a phase transfer catalyst and of inert organic solvents or mixtures thereof, and subsequently the further reaction is carried out within the temperature range from - 30°C to +120°C with catalytic to molar amounts of sulphuric acid and/or sulphonic acid.

2. Process according to Claim 1, characterised in that the reaction is carried out as a single-vessel reaction.

3. Process according to Claims 1 and 2, characterised in that the phase transfer catalyst used is benzyltriethylammonium chloride.

4. Process according to Claims 1 and 3, characterised in that the tert.-butyl ester of acemetacin is isolated as an intermediate product and the cleavage is carried out afterwards.

5. Process according to Claims 1 to 4, characterised in that the cleavage to give acemetacin is carried out with catalytic to molar amounts of sulphuric acid in glacial acetic acid or acetone or in mixtures of these solvents.

6. Process according to Claims 1 to 5, characterised in that the cleavage with sulphonic acids is carried out in

solvents which contain acetic acid or are organic.

## Revendications

1. Procédé pour la fabrication d'acémétacine, caractérisé en ce que l'on fait réagir une indométacine de formule générale II

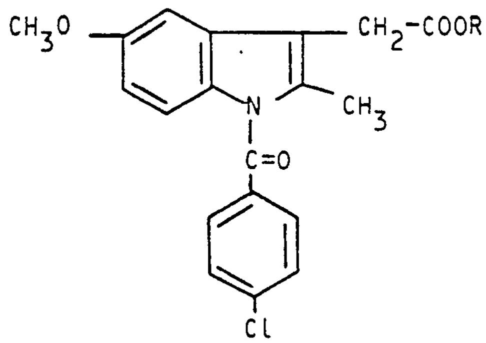

(II)

dans laquelle
R représente un cation de métal alcalin ou alcalino-terreux où le groupe $MY_4$,
dans lequel
M représente l'azote ou le phosphore et
Y représente l'hydrogène ou un groupe alkyle, cycloalkyle ou aryle éventuellement substitué, identique ou différent, avec un halogénoacétate de tertiobutyle de formule générale III

$Hal\text{-}CH_2\text{-}COO\text{-}C(CH_3)_3$ (III)

dans laquelle

Hal représente le chlore, le brome ou l'iode,
en présence d'un catalyseur de transfert de phase et de solvants organiques ou de leurs mélanges dans l'intervalle de températures de -30 à +70°C, et ensuite on met en oeuvre la réaction ultérieure avec des quantités catalytiques à molaires d'acide sulfurique et/ou d'acide sulfonique dans l'intervalle de températures de -30 à +120°C.

2. Procédé selon la revendication 1, caractérisé en ce que la réaction est effectuée comme réaction en un pot.

3. Procédé selon les revendications 1 et 2, caractérisé en ce que l'on utilise comme catalyseur de transfert de phase le chlorure de benzyltriéthylammonium.

4. Procédé selon les revendications 1 et 3, caractérisé en ce que l'on isole l'ester de tertiobutyle d'acémétacine comme produit intermédiaire et ensuite on met en oeuvre la dissociation.

5. Procédé selon les revendications 1 à 4, caractérisé en ce que la dissociation en acémétacine est mise en oeuvre avec des quantités catalytiques à molaires d'acide sulfurique dans l'acide acétique glacial ou l'acétone ou dans des mélanges de ces solvants.

6. Procédé selon les revendications 1 à 5, caractérisé en ce que la dissociation est mise en oeuvre avec des acides sulfoniques dans l'acide acétique ou des solvants organiques.